# FASCICULE DE BREVET EUROPEEN

(11) **EP 2 840 953 B1**
(45) Date de publication et mention de la délivrance du brevet: **30.05.2018**
(21) Numéro de dépôt: 13717530.3
(22) Date de dépôt: 23.04.2013
(51) Int. Cl.: A61B 5/00

(54) **SYSTEME D'IMAGERIE DE FLUORESCENCE POUR UN BLOC OPERATOIRE**
FLUORESZENZABBILDUNGSSYSTEM FÜR EINEN OPERATIONSSAAL
FLUORESCENCE IMAGING SYSTEM FOR AN OPERATING THEATRE

(30) Priorité: 25.04.2012 FR 1253786
(43) Date de publication de la demande: 04.03.2015
(73) Titulaire: Fluoptics, 38040 Grenoble (FR)
(72) Inventeur: RIZO, Philippe, F-38700 La Tronche (FR); MANGERET, Norman, F-38560 Jarrie (FR)
(74) Mandataire: Regimbeau
(86) Numéro de dépôt international: PCT/EP2013/058352
(87) Numéro de publication internationale: WO 2013/160279

(56) Documents cités:
- EP-A1- 2 071 322
- Sylvain Gioux ET AL: "Image-Guided Surgery using Invisible Near-Infrared Light: Fundamentals of Clinical Translation", NIH Public Access Author Manuscript, 1 octobre 2010 (2010-10-01), pages 1-31, XP055046858, Extrait de l'Internet: URL:http://www.ncbi.nlm.nih.gov/pmc/articl es/PMC3105445/pdf/nihms-294467.pdf [extrait le 2012-12-06] cité dans la demande
- SVEN D MIEOG J ET AL: "Toward Optimization of Imaging System and Lymphatic Tracer for Near-Infrared Fluorescent Sentinel Lymph Node Mapping in Breast Cancer", ANNALS OF SURGICAL ONCOLOGY, SPRINGER-VERLAG, NE, vol. 18, no. 9, 1 mars 2011 (2011-03-01), pages 2483-2491, XP019945153, ISSN: 1534-4681, DOI: 10.1245/S10434-011-1566-X cité dans la demande

## Description

### DOMAINE DE L'INVENTION

La présente invention concerne un système d'imagerie de fluorescence pour un bloc opératoire comprenant un dispositif d'éclairage de bloc opératoire et un dispositif d'imagerie médicale de fluorescence.

### ARRIERE PLAN DE L'INVENTION

L'imagerie médicale de fluorescence est une technique prometteuse notamment dans les interventions chirurgicales, en vue de guider l'action du chirurgien.

Cette technique repose sur l'administration au patient d'une substance comprenant un marqueur fluorescent, par exemple en vue d'observer un organe ou un tissu cible qui doit faire l'objet de l'intervention chirurgicale, ou en vue d'observer un écoulement suivi par le marqueur.

Grâce à la présence du marqueur fluorescent dans ou à proximité de l'organe ou le tissu cible, l'éclairement de la région du patient comprenant l'organe a pour effet d'exciter le marqueur, qui émet à son tour un rayonnement à une longueur d'onde légèrement supérieure à celle de la longueur d'onde d'excitation.

Les principales applications sont dans le domaine du proche infrarouge (NIR ou « Near InfraRed » selon la terminologie anglo-saxonne), c'est-à-dire que le rayonnement d'excitation et le rayonnement de fluorescence se situent dans la gamme de longueurs d'onde comprises entre 700 et 900 nm.

Une fois détecté, le rayonnement fluorescent peut être superposé à une image de l'organe concerné, pour visualiser l'organe ou le tissu cible par rapport à la partie externe visible de l'organe.

La Demanderesse fournit ainsi un dispositif d'imagerie médicale de fluorescence commercialisé sous la dénomination Fluobeam™.

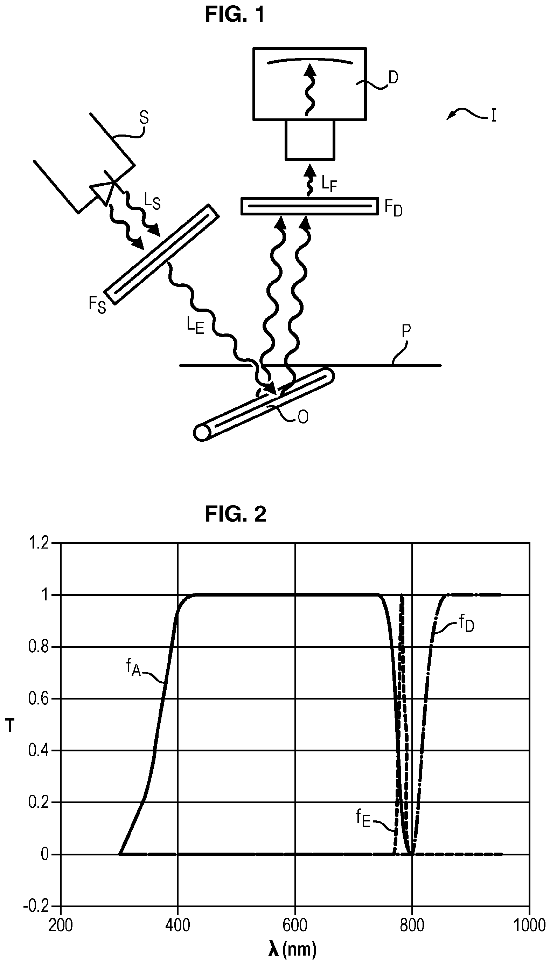

Le principe de l'imagerie de fluorescence est illustré de manière schématique à la figure 1.

Le dispositif d'imagerie I comprend une source lumineuse S destinée à exciter le marqueur fluorescent se trouvant une région O dont on souhaite obtenir des images (le marqueur pouvant être concentré dans cette région ou y passer en suivant un écoulement), le rayonnement L_{S} procuré par la source lumineuse ayant pour effet de rendre ledit marqueur fluorescent.

Dans les applications médicales, la région O est généralement située sous la peau P d'un patient.

Elle est rendue fluorescente par l'administration au patient d'une substance comprenant un marqueur fluorescent, de sorte que le marqueur vienne se concentrer ou passer dans la région O, et par l'excitation du marqueur par le rayonnement émis par la source lumineuse S.

Le dispositif I comprend par ailleurs un détecteur D adapté pour détecter et enregistrer le rayonnement fluorescent L_{F} émis par le marqueur situé dans la région O et excité par la source S.

Le détecteur comprend par exemple une caméra CCD.

A cet effet, la source lumineuse S est filtrée (filtre F_{S}) de sorte à exciter le marqueur fluorescent avec un rayonnement L_{E} ne contenant pas de longueurs d'onde correspondant à la fluorescence à mesurer.

Par exemple, lorsque la fluorescence à mesurer est dans le proche infrarouge (c'est-à-dire un domaine de longueurs d'onde comprises entre 700 et 900 nm), il est nécessaire de supprimer totalement les longueurs d'onde dans le domaine de fluorescence, non seulement dans le rayonnement d'excitation mais aussi dans la lumière ambiante.

En effet, dans le cas contraire, ces longueurs d'onde seraient détectées par le détecteur D et généreraient du bruit sur l'image de fluorescence, nuisant à la qualité de celle-ci.

En amont du détecteur D, le dispositif comprend un filtre F_{D} adapté pour ne laisser passer vers le détecteur que les photons L_{F} dont la longueur d'onde est celle de la fluorescence.

Pour la fluorescence dans le proche infrarouge, le filtre F_{D} est en général un filtre passe haut, qui transmet toutes les longueurs d'onde supérieures à un seuil donné.

La figure 2 illustre le principe de filtrage d'un tel dispositif d'imagerie de fluorescence et présente les courbes de transmission T en fonction de la longueur d'onde λ pour la lumière ambiante (courbe f_{A}), la source lumineuse d'excitation (courbe f_{E}), et le détecteur (courbe f_{D}).

Dans l'exemple illustré sur cette figure, la longueur d'onde d'excitation est de 780 nm, la lumière ambiante est filtrée de sorte à comprendre essentiellement des longueurs d'onde comprises entre 400 et 750 nm, tandis que le détecteur est filtré de sorte à recevoir toutes les longueurs d'onde supérieures à 820 nm environ.

Lors d'une intervention chirurgicale, le patient est installé sur une table dans un bloc opératoire et la zone à opérer est éclairée par un dispositif d'éclairage spécifique présent dans le bloc.

Un tel dispositif peut être par exemple un dispositif d'éclairage communément désigné par le terme « scialytique », se présentant sous la forme d'une coupole portée par un bras et orientée en direction du patient de sorte à éviter toute zone d'ombre.

Le dispositif d'imagerie de fluorescence décrit plus haut est également installé au bloc opératoire, et positionné de sorte à visualiser convenablement la région du patient dont on souhaite obtenir des images et à détecter la fluorescence émise par cette région.

La source lumineuse d'excitation peut par exemple être fixée au scialytique lui-même par un système de fixation approprié.

En raison de sa très forte puissance (typiquement, entre 40 et 150 kLux), l'éclairage de bloc opératoire est susceptible de perturber la détection du rayonnement de fluorescence en produisant des photons qui seront détectés par le détecteur.

Pour ne pas détériorer la qualité de l'image de fluorescence, il est nécessaire de filtrer la lumière fournie par le scialytique pour qu'elle ne contienne pas de longueurs d'onde correspondant à la fluorescence à mesurer.

Or, à 150 kLux, même une fuite minime de lumière provenant de l'éclairage du bloc opératoire dans la gamme de longueurs d'ondes mesurée par le détecteur à travers son filtre est susceptible de limiter la qualité des images de fluorescence.

Par ailleurs, les exigences en termes de qualité de l'éclairage opératoire sont drastiques.

On peut citer à cet égard la norme NF EN 60601-2-41 relative aux règles particulières de sécurité pour les éclairages chirurgicaux et les éclairages de diagnostic.

Ainsi, la lumière émise est de la lumière blanche qui doit présenter une température de couleur généralement comprise entre 3000 K et 6700 K.

En outre, la norme stipule que l'indice de rendu des couleurs (IRC) doit être compris entre 85 et 100%, de préférence de l'ordre de 95%.

Par conséquent, le filtrage de la lumière émise par le scialytique ne doit pas conduire à une dégradation des caractéristiques mentionnées ci-dessus.

Par ailleurs, en raison de la surface importante à filtrer pour un scialytique (de l'ordre de 0,5 m²), il est nécessaire de concevoir un filtre qui soit peu onéreux.

Des dispositifs d'imagerie de fluorescence ont déjà été décrits pour guider des interventions chirurgicales.

Certains systèmes, notamment le système PDE™ proposé par Hamamatsu et le système SPY™ proposé par Novadaq s'affranchissent de l'influence de la lumière blanche en éteignant le scialytique lors de la mise en oeuvre de l'imagerie de fluorescence.

Cependant, l'extinction de l'éclairage se prête mal à une intervention chirurgicale.

D'autres dispositifs d'imagerie de fluorescence ont été conçus pour fournir un éclairage en continu.

En particulier, le dispositif Flare™, ainsi que sa variante Mini-Flare™, ont fait l'objet de plusieurs publications.

Pour une utilisation en bloc opératoire, la lumière blanche émise par le scialytique ou le dispositif d'imagerie est filtrée à partir de la longueur d'onde d'excitation, de sorte à ne pas contenir de photons d'une longueur d'onde supérieure à la longueur d'onde d'excitation, qui, pour les dispositifs Flare™ et Mini-Flare™ peuvent être de 670 ou 760 nm [1]. La référence [1] décrit un système tel que défini dans le préambule de la revendication indépendante 1. Le principe de filtrage du système d'éclairage adapté au dispositif Flare™ est décrit dans [2].

Le détecteur de la fluorescence à 700 nm est filtré avec un filtre passe-bande de 689 à 725 nm, tandis que le détecteur de la fluorescence à 800 nm est filtré avec un filtre passe-bande de 800 à 848 nm.

Ces filtres passe-bande sont destinés à ne permettre que la détection des longueurs d'onde correspondant au signal de fluorescence, en éliminant les longueurs d'onde supérieures.

Le principe de filtrage du système d'éclairage adapté au dispositif Mini-Flare™ est décrit dans [3].

Dans ce dispositif, un unique détecteur est destiné à détecter les longueurs d'onde de fluorescence autour de 700 nm et de 800 nm.

Le détecteur est filtré par un filtre double passe-bande, avec une première bande passante comprise entre 689 et 725 nm, et une seconde bande passante comprise entre 803 et 853 nm.

Les filtres passe-bande utilisés pour les dispositifs Flare™ et Mini-Flare™ sont commercialisés par la société Chroma sous la référence HQ 817/25.

Un dispositif concurrent, proposé par la société SurgOptics, est décrit dans [4].

La source lumineuse d'excitation est une diode laser à une longueur d'onde de 750 nm, tandis que l'éclairage du bloc opératoire est procuré par une lampe halogène émettant de la lumière blanche.

Un filtre passe-bande Chroma HQ 795/50 est placé en amont du détecteur pour éviter de transmettre à celui-ci des longueurs d'onde supérieures à la longueur d'onde de fluorescence.

Cependant, pour tous les dispositifs décrits ci-dessus, on peut observer sur les images obtenues un fond de fluorescence qui diminue le contraste avec la fluorescence émise par l'organe ou le tissu d'intérêt.

Les auteurs attribuent généralement ce phénomène à de l'autofluorescence des tissus.

Cependant, pour les longueurs d'onde du proche infrarouge considérées, l'autofluorescence est négligeable et ne peut expliquer à elle seule le fond de fluorescence observé.

Une autre hypothèse pour expliquer cette qualité insatisfaisante des images peut être un filtrage insuffisant de la source lumineuse d'excitation.

En tout état de cause, il subsiste donc un besoin pour améliorer la qualité des images de fluorescence dans un contexte de bloc opératoire.

Un but de l'invention est donc de proposer un système d'éclairage et de filtrage de bloc opératoire adapté à la mise en oeuvre d'imagerie de fluorescence, et qui permette notamment d'optimiser la qualité de l'image de fluorescence, même en présence d'un éclairement intense et continu du champ opératoire.

Un autre but de l'invention est de proposer un système de filtrage qui puisse être aisément adapté aux équipements d'éclairage de bloc opératoire existants et à un coût modéré.

### BREVE DESCRIPTION DE L'INVENTION

A cet effet, il est proposé un système d'imagerie de fluorescence pour un bloc opératoire comprenant un dispositif d'éclairage de bloc opératoire apte à émettre une lumière blanche et un dispositif d'imagerie de fluorescence,
ledit dispositif d'imagerie de fluorescence comprenant :
- une source lumineuse apte à émettre un rayonnement d'excitation d'un marqueur fluorescent dans une gamme de longueurs d'onde d'émission comprise entre 600 et 900 nm,
- un détecteur adapté pour détecter le rayonnement fluorescent émis par ledit marqueur sous l'effet d'une excitation par ladite source lumineuse,
- un filtre du détecteur conprenant un filtre passe haut ou passe bande adapté pour bloquer le rayonnement d'excitation et transmettre au détecteur les photons dont la longueur d'onde est incluse dans la gamme de longueurs d'onde du rayonnement fluorescent émis par ledit marqueur, Le système d'imagerie comprenant par ailleurs un filtre passe bas placé devant le dispositif d'éclairage et dont la longueur d'onde de coupure est inférieure à la gamme d'émission du marqueur fluorescent, ledit filtre passe-bas pouvant néanmoins présenter une remontée ou des fluctuations de l'atténuation pour des longueurs d'onde supérieures à la gamme d'émission du marqueur fluorescent.

Conformément à l'invention, le filtre du détecteur comprend un filtre passe bas combiné au filtre passe haut ou passe bande de telle sorte que, dans une gamme de longueurs d'onde s'étendant à partir d'une longueur d'onde de coupure supérieure du filtre du détecteur inférieure à la longueur d'onde à partir de laquelle on observe une remontée ou des fluctuations de l'atténuation du filtre passe bas du dispositif d'éclairage, le produit de l'atténuation du filtre du détecteur et de l'atténuation du filtre passe-bas du dispositif d'éclairage conduit à une atténuation par un facteur d'au moins 10⁶.

Dans le présent texte, le terme « filtre » peut englober un filtre élémentaire ou une combinaison de filtres.

Comme expliqué en détail plus bas, ce filtrage du détecteur a pour effet de supprimer un biais et du bruit de fond dus à la remontée du filtre passe-bas du dispositif d'éclairage qui laisse passer une lumière parasite suffisamment intense, du fait de l'intensité de l'éclairage du bloc opératoire, pour masquer une partie des photons de fluorescence provenant des zones profondes, et qui sont par conséquent peu nombreux.

Grâce à ce filtrage, le détecteur est plus sensible au signal de fluorescence provenant des couches profondes, et produit des images plus contrastées.

Par ailleurs, dans la mesure où ce filtrage est appliqué au détecteur, qui présente une faible surface, sa mise en oeuvre ne grève pas le coût du système.

Selon un mode de réalisation de l'invention, la longueur d'onde du rayonnement d'excitation est comprise entre 630 et 810 nm.

De manière préférée, la largeur de la bande dans laquelle le filtre du détecteur transmet le rayonnement fluorescent est comprise entre 50 et 70 nm.

Selon une forme d'exécution particulièrement avantageuse de l'invention, la gamme de longueurs d'onde dans laquelle le produit de l'atténuation du filtre du détecteur et de l'atténuation du filtre passe-bas du dispositif d'éclairage conduit à une atténuation par un facteur d'au moins 10⁶ s'étend au moins jusqu'à la longueur d'onde limite de détection du détecteur.

Ainsi, la gamme de longueurs d'onde dans laquelle le produit de l'atténuation du filtre du détecteur et de l'atténuation du filtre passe-bas du dispositif d'éclairage conduit à une atténuation d'un facteur au moins 10⁶ s'étend de préférence au moins jusqu'à 1000 nm, et de manière encore préférée jusqu'à 1150 nm.

Par ailleurs, dans la gamme de longueurs d'onde transmises par le filtre du détecteur, le filtre passe bas du dispositif d'éclairage présente une atténuation par un facteur d'au moins 10⁶.

Le dispositif d'éclairage peut comprendre une coupole d'éclairage positionnable par un bras articulé.

De manière alternative, le dispositif d'éclairage peut comprendre une lampe frontale destinée à être placée sur la tête du chirurgien.

Selon un mode de réalisation, le dispositif d'éclairage est adapté pour fournir un éclairage continu.

Selon un autre mode de réalisation, le dispositif d'éclairage est adapté pour fournir un éclairage pulsé.

De préférence, le dispositif d'éclairage comprend des diodes électroluminescentes.

Par ailleurs, la puissance du dispositif d'éclairage est avantageusement supérieure ou égale à 40 kLux.

Le détecteur peut être une caméra CCD ou CMOS.

Selon un mode de réalisation particulier, la longueur d'onde d'excitation est de 780 nm et le filtre du détecteur transmet le rayonnement dans une bande comprise entre 820 et 850 nm.

Selon un autre mode de réalisation, la longueur d'onde d'excitation est de 750 nm et le filtre du détecteur transmet le rayonnement dans une bande comprise entre 780 et 870 nm.

### BREVE DESCRIPTION DES DESSINS

D'autres caractéristiques et avantages de l'invention ressortiront de la description détaillée qui va suivre, en référence aux dessins annexés sur lesquels :
- la figure 1 est un schéma de principe de l'imagerie de fluorescence,
- la figure 2 est un graphique présentant le principe du filtrage de la lumière ambiante, de la lumière d'excitation et du détecteur dans le cadre de l'imagerie de fluorescence,
- la figure 3 est un schéma de principe d'un système d'imagerie selon l'invention,
- les figures 4A et 4B présentent respectivement les spectres d'éclairage de bloc opératoire par des lampes halogènes et par des diodes électroluminescentes,
- la figure 5 illustre la courbe de transmission d'un filtre passe bas utilisé dans des éclairages de bloc opératoire existants et présentant des rebonds de l'atténuation entre 800 et 900 nm,
- la figure 6 illustre la courbe de transmission d'un filtre passe bas pouvant être utilisé pour filtrer l'éclairage du bloc opératoire,
- les figures 7A et 7B illustrent les courbes de transmission de filtres passe bande employés pour filtrer le détecteur de certains dispositifs d'imagerie de fluorescence du marché,
- la figure 8A illustre la courbe de sensibilité d'un capteur CCD du détecteur en fonction de la longueur d'onde,
- la figure 8B illustre la courbe de l'efficacité quantique d'un capteur CMOS du détecteur en fonction de la longueur d'onde,
- les figures 9a à 9c illustrent respectivement les courbes de transmission du filtre passe bas de l'éclairage de bloc opératoire, du filtre passe bas du détecteur selon un mode de réalisation de l'invention, et du filtre passe bande du détecteur,
- les figures 10A et 10B sont des images de fluorescence obtenues respectivement avec un système de filtrage du détecteur selon l'état de la technique et avec un système conforme à l'invention,
- la figure 11 présente des histogrammes obtenus respectivement avec un système de filtrage du détecteur selon l'état de la technique et avec un système conforme à l'invention.

### DESCRIPTION DETAILLEE DE L'INVENTION

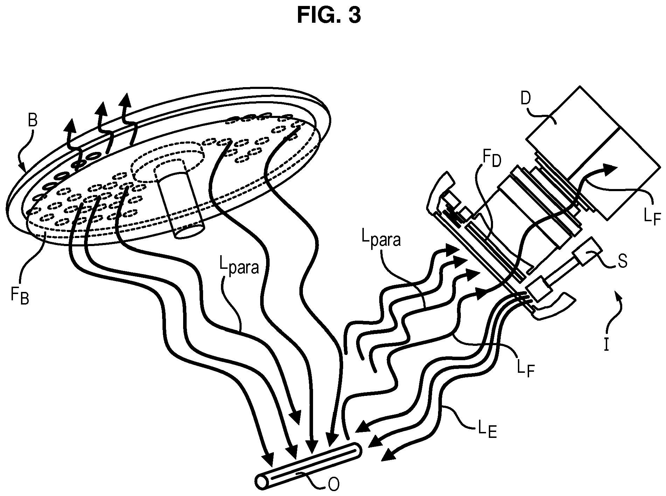

La figure 3 est un schéma d'ensemble du système selon un mode de réalisation de l'invention.

Ce système comprend un dispositif B d'éclairage de bloc opératoire et un dispositif I d'imagerie de fluorescence, orientés vers une région O qui symbolise une partie d'un patient (être humain ou animal) dont on souhaite obtenir des images de fluorescence, et dans laquelle on injecte à cet effet un marqueur fluorescent.

Ledit dispositif I d'imagerie de fluorescence est avantageusement un dispositif commercialisé par la Demanderesse sous le nom de Fluobeam™.

Ce dispositif I comprend une source lumineuse S apte à émettre un rayonnement d'excitation L_{E} du marqueur fluorescent situé ou passant dans la région O à observer dans une gamme de longueurs d'onde d'émission L_{F} comprise entre 600 et 900 nm, et un détecteur D adapté pour détecter le rayonnement fluorescent L_{F} émis par ledit marqueur sous l'effet d'une excitation par ladite source lumineuse S.

Pour effectuer la mesure de fluorescence, on filtre le détecteur par un filtre F_{D} qui élimine les longueurs d'onde du rayonnement visible, qui correspondent à l'éclairage du bloc opératoire, tout en laissant passer les longueurs d'onde du proche infra-rouge correspondant à la fluorescence.

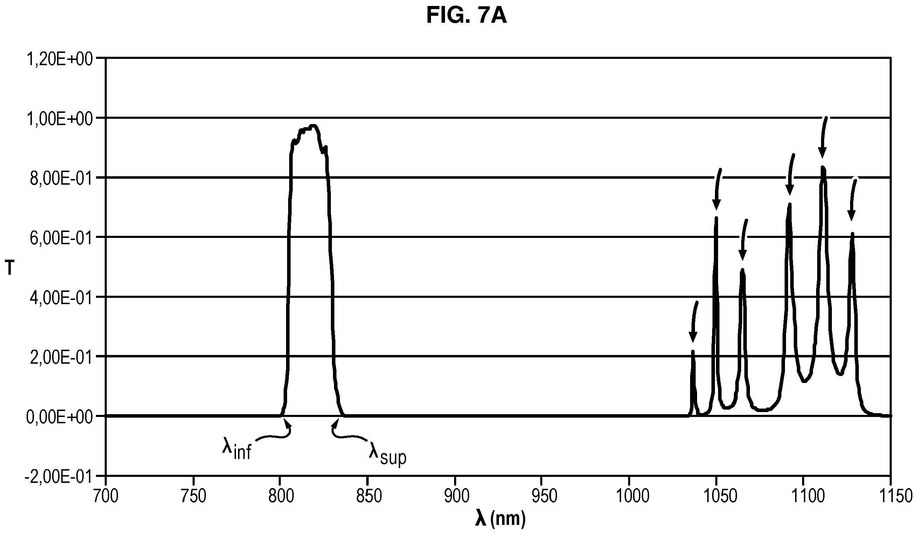

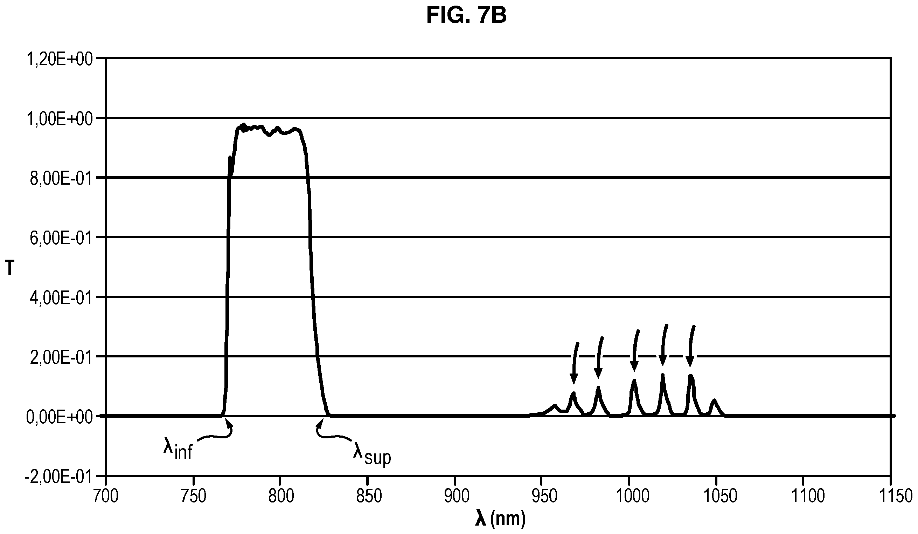

Selon un mode de réalisation préféré, le filtre du détecteur comprend un filtre passe bande F_{D1} dont la bande passante est située dans les longueurs d'onde du proche infra-rouge correspondant à la fluorescence. Un exemple de courbe de transmission d'un tel filtre passe bande est présenté sur les figures 7A et 7B, qui sont commentées plus bas.

De manière alternative, le filtre du détecteur peut transmettre également les longueurs d'onde inférieures au rayonnement visible sans pour autant sortir du cadre de la présente invention. Cependant, dans un objectif de concision, on pourra employer dans la suite du texte le terme « passe bande » pour ces deux variantes.

Le filtre F_{D} peut ainsi être constitué d'une combinaison de filtres permettant d'obtenir les plages d'atténuation et de transmission souhaitées.

Selon une forme d'exécution de l'invention illustrée sur la figure 3, le dispositif d'éclairage de bloc opératoire est un scialytique, c'est-à-dire une source lumineuse de grande dimension destinée à éclairer le champ opératoire avec une lumière blanche en évitant toute zone d'ombre constituée par exemple par la tête et les mains du chirurgien, les instruments employés, etc.

Il s'agit d'une source lumineuse très puissante (40 kLux ou davantage, souvent jusqu'à 150 kLux).

La norme NF EN 60601-2-41 citée plus haut impose par ailleurs le respect d'une certaine gamme de température de couleur, ainsi que d'une certaine gamme d'indice de rendu des couleurs (IRC).

A l'heure actuelle, la température de couleur doit généralement être comprise entre 3000 K et 6700 K et l'indice de rendu des couleurs doit être compris entre 85 et 100%, de préférence de l'ordre de 95%.

Le scialytique se présente typiquement sous la forme d'une coupole comprenant une pluralité de lampes.

Cette coupole est généralement solidaire d'un bras articulé fixé au plafond ou à tout support approprié du bloc opératoire, de manière à pouvoir être orientée vers la zone d'intervention pour fournir au chirurgien un éclairage avec le meilleur contraste possible.

Selon une autre forme d'exécution de l'invention (non illustrée), le dispositif d'éclairage de bloc opératoire est une lampe frontale qui est destinée à être placée sur la tête du chirurgien.

Hormis le fait que le faisceau lumineux soit plus focalisé avec une telle lampe frontale qu'avec un scialytique, les contraintes liées à la qualité de l'éclairage (notamment en termes de puissance, de température de couleur et d'indice de rendu des couleurs) sont les mêmes pour ces deux types de dispositifs.

Par conséquent, la solution de filtrage selon l'invention et qui est décrite en détail plus bas s'applique pour tout dispositif d'éclairage de bloc opératoire, qu'il s'agisse d'un scialytique ou d'une lampe frontale.

Les éclairages de bloc opératoire actuels comprennent soit des lampes halogènes, soit des diodes électroluminescentes (LEDs), ces dernières tendant à remplacer les premières.

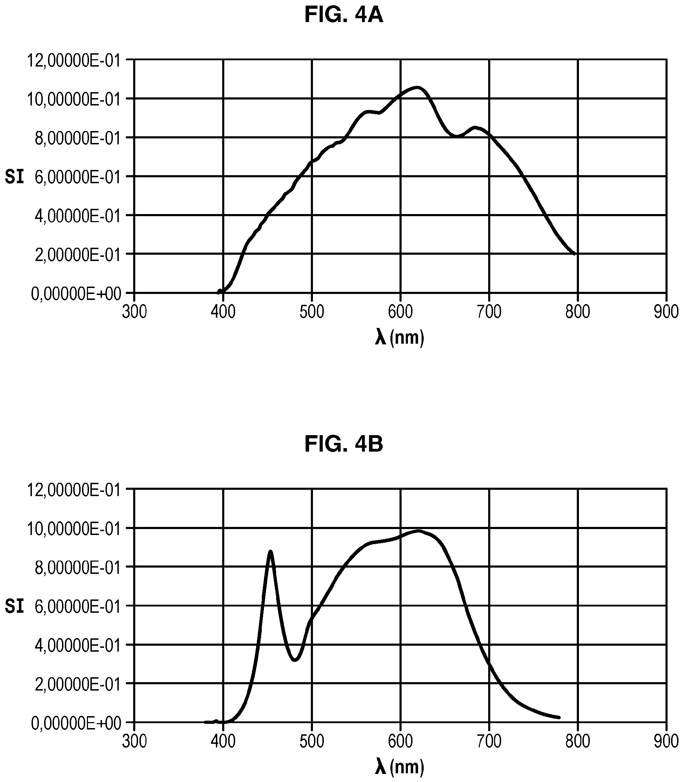

La figure 4A présente le spectre d'un éclairage de type halogène, qui présente un indice de rendu des couleurs de 91,5, un flux lumineux par unité de surface de 66 kLux et une température de couleur de 4000 K.

La figure 4B présente le spectre d'un éclairage à diodes électroluminescentes (LEDs), qui présente un indice de rendu des couleurs de 90,5, un flux lumineux par unité de surface de 60 kLux et une température de couleur de 3719 K.

La comparaison de ces deux spectres montre que l'éclairage à LEDs fournit un niveau de lumière pour les longueurs d'onde supérieures à 700 nm beaucoup plus faible que l'éclairage halogène.

Il est donc avantageux, pour mettre en oeuvre l'imagerie de fluorescence dans le proche infra-rouge en bloc opératoire, de choisir un éclairage à LEDs, celui-ci permettant d'éliminer plus facilement la composante infra-rouge.

Pour éviter que les longueurs d'onde infra-rouge du dispositif d'éclairage ne soient transmises, on place devant celui-ci un filtre passe-bas F_{B}, dont la fréquence de coupure est de l'ordre de 700 à 750 nm.

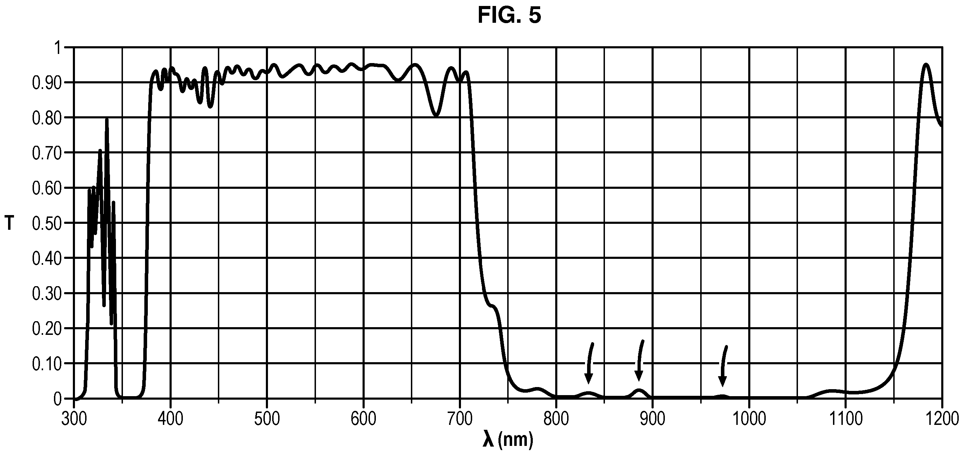

La figure 5 présente le spectre d'un filtre interférentiel passe bas communément utilisé pour couper le proche infrarouge de l'éclairage de bloc opératoire.

En raison de la grande surface du dispositif d'éclairage (typiquement, de l'ordre de 0,5 m²), la solution technique la plus raisonnable économiquement pour le filtrage de l'éclairage de bloc opératoire est l'utilisation d'un filtre interférentiel à bas coût.

Cependant, sur ce type de filtre, on observe des rebonds (désignés par les flèches) de l'atténuation entre 800 et 900 nm, qui engendrent de la lumière parasite dans les longueurs d'onde de fluorescence.

Un tel filtre n'est donc pas utilisable pour l'application visée.

On choisit donc un filtre interférentiel plus performant dans la gamme des longueurs d'onde de fluorescence, qui permet de filtrer l'éclairage du scialytique ou de la lampe frontale de manière très franche dans une gamme de longueur d'ondes au-dessus de 700 ou 750 nm, afin de ne pas détériorer l'indice de rendu des couleurs ou tout au moins de pouvoir le restaurer en ajoutant une composante rouge.

L'homme du métier sera à même de choisir, parmi les filtres commercialisés du marché, un filtre présentant les performances requises, ou de faire fabriquer par une société spécialisée le filtre adéquat sur la base d'un cahier des charges.

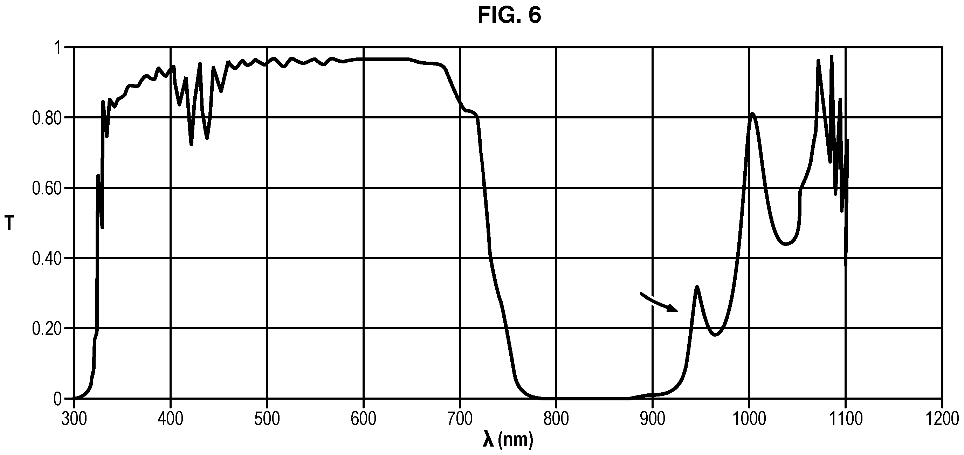

La figure 6 présente le spectre d'un filtre interférentiel pouvant être utilisé pour filtrer le scialytique ou la lampe frontale pour la mise en oeuvre de l'invention.

Celui-ci présente une bonne atténuation entre 800 et 900 nm, mais une remontée significative (désignée par les flèches) de l'atténuation au-dessus de 900 nm.

Quand on filtre le scialytique ou la lampe frontale par ce type de filtre, il existe donc, dans le spectre d'éclairement, de la lumière parasite dans les grandes longueurs d'onde du proche infra-rouge, c'est-à-dire autour de 900 nm.

Cette lumière parasite est désignée par le repère Lₚₐᵣₐ sur la figure 3.

Comme on le verra plus bas, cette remontée peut être compensée par un filtrage passe bas du détecteur coupant les longueurs d'onde élevées correspondantes.

Par ailleurs, de manière connue, le filtrage du détecteur D comprend généralement un filtre passe bande F_{D1} destiné à ce que seuls les photons correspondant à la fluorescence L_{F} émise par le marqueur soient détectés.

D'une manière générale, une largeur de bande passante de l'ordre de 50 à 70 nm est appropriée, la valeur des longueurs d'onde de coupure inférieure et supérieure (notées respectivement λ_{inf} et λₛᵤₚ) étant choisie en fonction du spectre d'émission de fluorescence du marqueur considéré.

Or, les inventeurs ont constaté que les filtres passe-bande utilisés dans les dispositifs du marché présentaient en réalité une remontée ou des rebonds de l'atténuation pour les longueurs d'onde comprises entre 800 et 1000 nm.

La figure 7A présente ainsi le spectre d'un filtre Chroma HQ817-25 qui est utilisé par exemple dans les systèmes Flare™ et Mini-Flare™ décrits plus haut.

On observe une très bonne atténuation entre 850 et 1040 nm, mais des rebonds importants (désignés par les flèches) à partir de 1050 nm.

La figure 7B présente quant à elle le spectre d'un filtre Chroma HQ795/50 qui est utilisé par exemple dans le système SurgOptix mentionné plus haut.

On observe une très bonne atténuation entre 830 et 940 nm, mais des rebonds significatifs (désignés par les flèches) entre 950 et 1050 nm.

Il n'est pas usuel de s'intéresser au comportement des filtres dans les longueurs d'onde supérieures à 900 nm.

En effet, on considère habituellement que pour ces longueurs d'onde, la sensibilité des détecteurs (capteurs CCD ou CMOS) est faible et que les LEDs de l'éclairage du bloc opératoire n'émettent plus.

En outre, dans les applications traditionnelles de fluorescence, l'éclairage ambiant est relativement peu puissant, de sorte que la lumière parasite pour de telles longueurs d'onde influe peu sur le signal mesuré par le détecteur.

Cependant, les inventeurs ont constaté que le détecteur présentait encore une certaine sensibilité jusqu'à 1000 nm, voire plus.

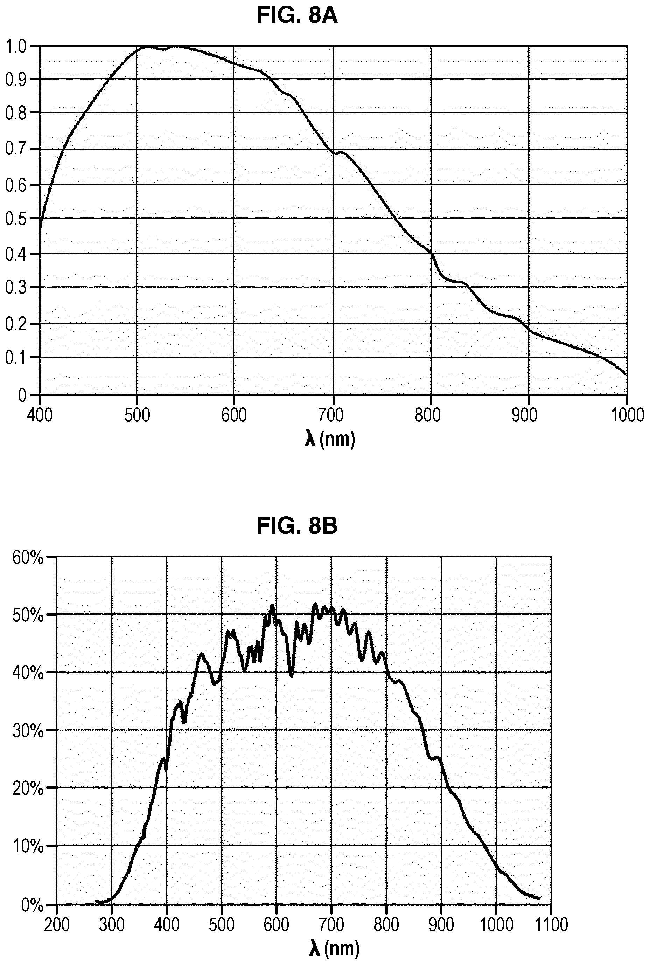

La figure 8A illustre ainsi la sensibilité en fonction de la longueur d'onde d'un capteur CCD typiquement utilisé dans un dispositif d'imagerie de fluorescence tel que le dispositif Fluobeam™.

La figure 8B illustre l'efficacité quantique (QE) en fonction de la longueur d'onde d'un capteur CMOS, qui pourrait également être utilisé dans le dispositif d'imagerie de fluorescence.

Ces types de capteurs présentent une sensibilité élevée pour les longueurs d'onde visibles et une sensibilité plus faible mais encore significative pour les longueurs d'onde correspondant au proche infrarouge.

Bien que cette sensibilité soit faible, dans la mesure où l'éclairage procuré par le dispositif du bloc opératoire est très puissant (pouvant atteindre 150 kLux), la lumière parasite qui résulte de la mauvaise atténuation par le filtre passe-bas du scialytique ou de la lampe frontale et le filtre passe-bande du détecteur est suffisamment importante pour produire un bruit de fond significatif dans le signal de fluorescence mesuré par le capteur.

Partant de ces considérations, une approche intuitive consisterait à améliorer le filtrage du dispositif d'éclairage de bloc opératoire.

On peut en effet considérer que, s'il était correctement mis en oeuvre, un tel filtrage permettrait de supprimer à la source tous les photons parasites. Ainsi, le détecteur ne détecterait que les photons de fluorescence, notamment ceux correspondant aux longueurs d'onde les plus élevées.

Cependant, comme indiqué plus haut, au regard de la surface du dispositif d'éclairage de bloc opératoire (de l'ordre de 0,5 m² pour un scialytique), le coût d'un filtre adéquat serait prohibitif.

Par ailleurs, même si cette solution permettrait de détecter davantage de photons, ceux-ci sont peu nombreux et la sensibilité du détecteur aux longueurs d'onde correspondantes est faible.

Enfin, l'ajout d'un filtre supplémentaire risquerait de modifier l'indice de rendu des couleurs et la température de couleur de l'éclairage de bloc opératoire.

Les inventeurs ont au contraire choisi d'appliquer un filtrage supplémentaire au détecteur, malgré la perte de photons détectables par le détecteur, allant ainsi à l'encontre du préjugé selon lequel un filtre additionnel conduirait à diminuer la qualité du signal mesuré par le détecteur.

On pourrait en effet craindre, en filtrant davantage le détecteur, de ne pas détecter suffisamment de photons pour fournir des images suffisamment contrastées.

Le filtrage additionnel du détecteur consiste en un filtre passe bas qui, au-dessus de la longueur d'onde de coupure, présente une atténuation très forte.

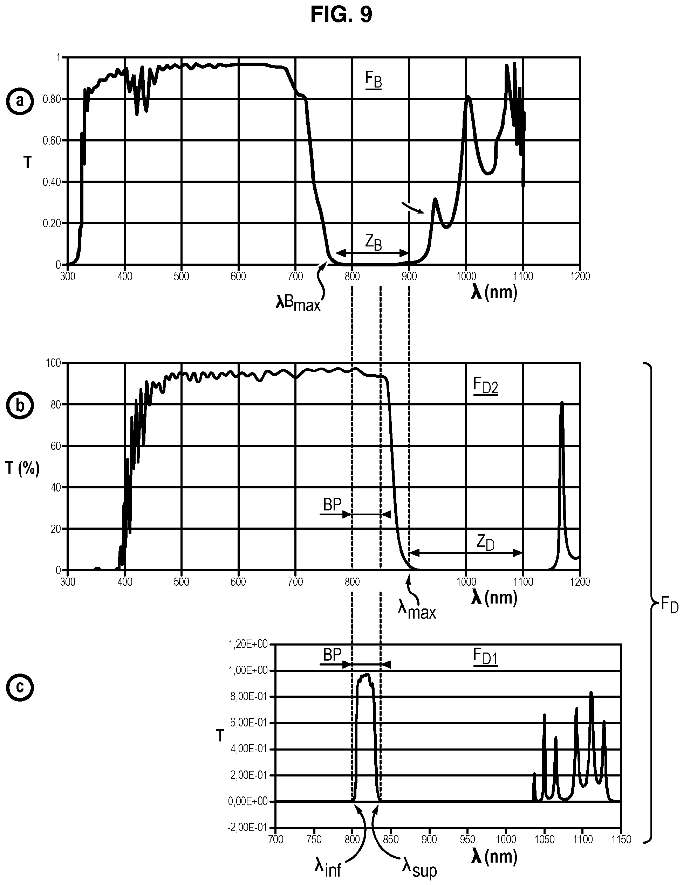

En d'autres termes, le filtre F_{D} du détecteur D comprend un filtre passe bande ou passe haut F_{D1} (cf. figure 9c) combiné à un filtre passe bas F_{D2} (cf. figure 9b) qui transmet les longueurs d'onde du proche infrarouge devant être détectées par le détecteur et dans lequel, au-dessus de sa longueur d'onde de coupure supérieure λₘₐₓ, le produit des atténuations du filtre du dispositif d'éclairage du bloc opératoire et du filtre du détecteur conduit à une atténuation d'au moins un facteur 10⁶.

Cette condition doit de préférence être respectée au moins jusqu'à la limite de détection du détecteur D.

La gamme de longueurs d'onde concernée par cette atténuation drastique est schématisée par la zone Z_{D} sur la figure 9b, qui présente simultanément les courbes de transmission du filtre de l'éclairage de bloc opératoire (figure 9a, correspondant à la figure 6 commentée plus haut) et d'un exemple de filtre passe bas additionnel F_{D2} approprié pour le détecteur (figure 9b), et d'un exemple de filtre passe bande F_{D1} du détecteur (figure 9c, correspondant à la figure 7A commentée plus haut).

Naturellement, les courbes de transmission présentées ici le sont à titre purement illustratif, et l'homme du métier pourra faire varier les caractéristiques de transmission et les longueurs d'onde de coupure en fonction des spécificités de l'éclairage de bloc opératoire et du dispositif d'imagerie considérés.

Dans le choix du filtre du dispositif d'éclairage de bloc opératoire, il convient en outre de veiller à ce que le filtre passe bas F_{B} du scialytique ou de la lampe frontale ne transmette aucune longueur d'onde devant être mesurée par le détecteur (en d'autres termes, la longueur d'onde de coupure λ_{Bmax} du filtre passe bas F_{B} est inférieure à la longueur d'onde de coupure basse λ_{inf} du filtre passe bande F_{D1} dont la bande passante BP est comprise dans la zone Z_{B} sur la figure 9a, correspondant à une atténuation de l'éclairage de bloc opératoire par un facteur d'au moins 10⁶, de préférence d'au moins 10⁷) et à ce que les remontées ou fluctuations de l'atténuation du filtre passe bas F_{B} du scialytique ou de la lampe frontale n'interviennent que pour des longueurs d'onde situées au-dessus de la bande passante BP du filtre du détecteur.

Comme illustré aux figures 9a et 9b, la remontée du filtre passe bas F_{B} du scialytique ou de la lampe frontale débute à partir d'une longueur d'onde située au-dessus de la bande passante BP du détecteur, mais, comme elle est située au-dessus de la fréquence de coupure λₘₐₓ, cette remontée est atténuée par le filtre F_{D2} (zone ZD sur la figure 9b).

Sur la figure 9b, on constate que le filtre supplémentaire F_{D2} du détecteur présente une remontée importante vers 1200 nm ; cependant, cette longueur d'onde est supérieure à la longueur d'onde limite de détection du détecteur, de sorte que cette remontée n'a pas d'incidence sur la qualité des images.

L'homme du métier sera à même de choisir, parmi les filtres commercialisés du marché, un filtre présentant les performances requises, ou de faire fabriquer par une société spécialisée le filtre adéquat sur la base d'un cahier des charges de filtrage du détecteur.

Les inventeurs ont vérifié que, de manière surprenante, la qualité des images et la séparation entre les différentes structures étaient nettement améliorées avec ce filtrage supplémentaire du détecteur.

En particulier, ils ont observé que celui-ci permettait de supprimer un biais et du bruit de fond qui masquaient les photons de fluorescence provenant des zones profondes, et qui sont par conséquent peu nombreux.

Le détecteur ainsi filtré est alors plus sensible au signal de fluorescence provenant des couches profondes, et produit des images plus contrastées.

Par ailleurs, dans la mesure où la surface du détecteur est beaucoup plus faible que celle du scialytique, l'emploi d'un filtre très performant pour obtenir une atténuation d'un facteur supérieur à 10⁶ dans les longueurs d'ondes élevées ne pénalise pas économiquement le système.

A titre d'illustration non limitative, le dispositif Fluobeam™ commercialisé par la Demanderesse présente, dans un premier mode de réalisation, une longueur d'onde d'excitation de 780 nm et une bande passante du filtre passe-bande F_{D} comprise entre 820 et 850 nm ; dans un second mode de réalisation, la longueur d'onde d'excitation est de 750 nm et la bande passante du filtre passe-bande F_{D} est comprise entre 780 et 870 nm.

Naturellement, les valeurs numériques données plus haut le sont à titre purement illustratif et l'homme du métier sera en mesure, en fonction des filtres à sa disposition et des longueurs d'onde de fluorescence à détecter, de mettre au point les filtres adéquats pour l'éclairage du bloc opératoire et le détecteur.

### Résultats expérimentaux

Une intervention chirurgicale a été effectuée sur le coeur d'un animal, en utilisant le système décrit plus haut, dans les mêmes conditions expérimentales, à l'exception, dans le deuxième cas, de l'ajout du filtrage additionnel du détecteur décrit plus haut (dont la courbe de transmission est donnée à la figure 9b).

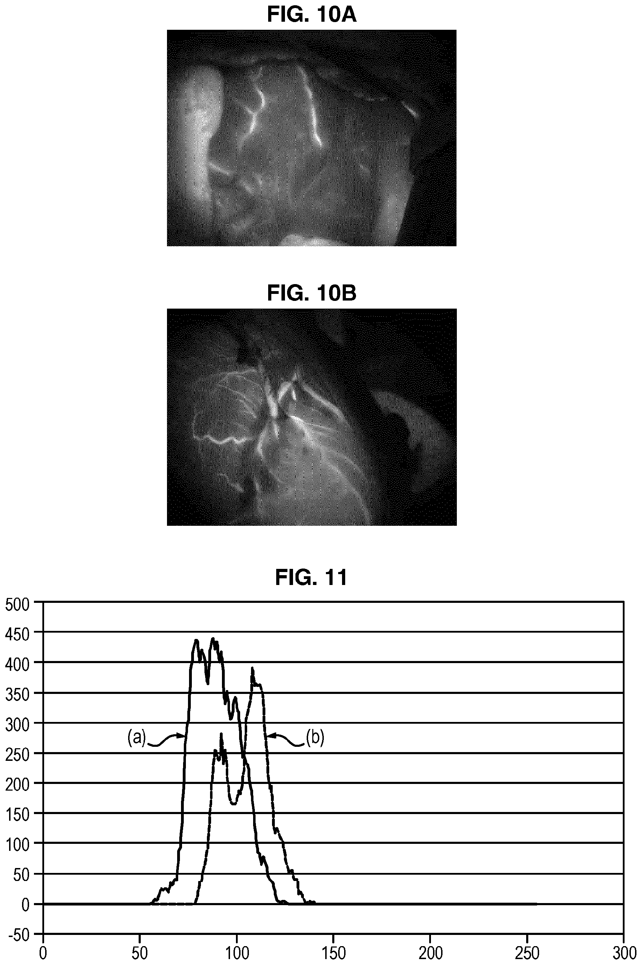

Les figures 10A et 10B sont des images de fluorescence du coeur enregistrées après injection de vert d'indocyanine (ICG), respectivement sans et avec le filtre passe bas supplémentaire du détecteur.

On observe qu'avec le système conforme à l'invention, on voit beaucoup plus de vaisseaux sanguins situés en profondeur.

Ce résultat peut également être observé sur la figure 11, qui présente un histogramme obtenu dans une zone hors coronaires, sans (courbe (a)) et avec (courbe (b)) le filtre passe bas supplémentaire du détecteur.

Sur cet histogramme, l'un des modes correspond à la surface du coeur et l'autre aux vaisseaux profonds.

La comparaison de ces deux courbes montre clairement que l'aspect bimodal de l'histogramme est beaucoup plus fort sur l'image acquise avec le filtre passe bas supplémentaire du détecteur que sur celle acquise sans ce filtre.

Il en résulte que le filtre additionnel augmente sensiblement le contraste des vaisseaux profonds.

### REFERENCES

[1] S. Gioux, H. S. Choi et J. F. Frangioni, «Image-Guided Surgery Using Invisible Near-Infrared Light: Fundamentals of Clinical Translation,» Molecular Imaging, vol. 9, n° 15, pp. 237-255.
[2] S. L. Troyan, V. Kianzad, S. L. Gibbs-Strauss, S. Gioux, A. Matsui, R. Oketokoun, L. Ngo, A. Khamene, F. Azar et J. V. Frangioni, «The FLARE(TM) Intraoperative Near-Infrared Fluorescence Imaging System: A First-in-Human Clinical Trial in Breast Cancer Sentinel Lymph Node Mapping,» Ann Surg Oncol, vol. 16, pp. 2943-2952, 2009.
[3] J. S. D. Mieog, S. S. Troyan, M. Hutteman, K. J. Donohoe, J. R. van der Vorst, A. Stockdale, G.-J. Liefers, H. S. Choi, S. L. Gibbs-Strauss, H. Putter, S. Gioux, P. J. Kuppen, Y. Ashitate, C. W. Löwik, V. T. Smit, R. Oketokoun, L. H. Ngo, C. J. van de Velde, J. V. Frangioni et A. L. Vahrmeijer, «Toward Optimization of Imaging System and Lymphatic Tracer for Near-Infrared Fluorescent Sentinel Lymph Node Mapping in Breast Cancer,» AnnSurg Oncol, vol. 18, pp. 2483-2491, 2011.
[4] G. Themelis, J. S. Yoo, K.-S. Soh, R. Schulz et V. Ntziachristos, «Real-time intraoperative fluorescence imaging system using light-absorption correction,» Journal of Biomedical Optics, vol. 14, n° %16, 2009.

## Revendications

1. Système d'imagerie de fluorescence pour un bloc opératoire comprenant un dispositif (B) d'éclairage de bloc opératoire apte à émettre une lumière blanche et un dispositif (I) d'imagerie de fluorescence,
ledit dispositif (I) d'imagerie de fluorescence comprenant :
- une source lumineuse (S) apte à émettre un rayonnement d'excitation (L_{E}) d'un marqueur fluorescent dans une gamme de longueurs d'onde d'émission (L_{F}) comprise entre 600 et 900 nm,
- un détecteur (D) adapté pour détecter le rayonnement fluorescent (L_{F}) émis par ledit marqueur sous l'effet d'une excitation par ladite source lumineuse (S),
- un filtre (F_{D}) du détecteur (D) comprenant un filtre passe haut ou passe bande (F_{D1}) adapté pour bloquer le rayonnement d'excitation (L_{E}) et transmettre au détecteur (D) les photons dont la longueur d'onde est incluse dans la gamme de longueurs d'onde du rayonnement fluorescent (L_{F}) émis par ledit marqueur, le système comprenant par ailleurs un filtre passe bas (F_{B}) placé devant le dispositif d'éclairage (B) et dont la longueur d'onde de coupure λ_{Bmax}) est inférieure à la gamme d'émission (L_{F}) du marqueur fluorescent, ledit filtre passe-bas (F_{B}) pouvant néanmoins présenter une remontée ou des fluctuations de l'atténuation pour des longueurs d'onde supérieures à la gamme d'émission (L_{F}) du marqueur fluorescent,
le système étant **caractérisé en ce que** le filtre (F_{D}) du détecteur comprend un filtre passe bas (F_{D2}) combiné au filtre passe haut ou passe bande (F_{D1}) de telle sorte que, dans une gamme (Z_{D}) de longueurs d'onde s'étendant à partir d'une longueur d'onde de coupure supérieure (λₘₐₓ) inférieure à la longueur d'onde à partir de laquelle on observe une remontée ou des fluctuations de l'atténuation du filtre passe bas du dispositif d'éclairage, le produit de l'atténuation du filtre (F_{D}) du détecteur et de l'atténuation du filtre passe-bas (F_{B}) du dispositif d'éclairage conduit à une atténuation par un facteur au moins 10⁶.

2. Système selon la revendication 1, **caractérisé en ce que** la longueur d'onde du rayonnement d'excitation est comprise entre 630 et 810 nm.

3. Système selon l'une des revendications 1 ou 2, **caractérisé en ce que** la largeur de la bande dans laquelle le filtre du détecteur transmet le rayonnement fluorescent est comprise entre 50 et 70 nm.

4. Système selon l'une des revendications 1 à 3, **caractérisé en ce que** la gamme (Z_{D}) de longueurs d'onde dans laquelle le produit de l'atténuation du filtre du détecteur et de l'atténuation du filtre passe-bas du dispositif d'éclairage (B) conduit à une atténuation par un facteur au moins 10⁶ s'étend au moins jusqu'à la longueur d'onde limite de détection du détecteur (D).

5. Système selon l'une des revendications 1 à 4, **caractérisé en ce que** la gamme de longueurs d'onde dans laquelle le produit de l'atténuation du filtre du détecteur (D) et de l'atténuation du filtre passe-bas du dispositif d'éclairage (B) conduit à une atténuation par un facteur au moins 10⁶ s'étend au moins jusqu'à 1000 nm, de préférence jusqu'à 1150 nm.

6. Système selon l'une des revendications 1 à 5, **caractérisé en ce que** dans la gamme (λ_{inf}, λₛᵤₚ) de longueurs d'ondes transmise par le filtre du détecteur, le filtre passe bas (F_{B}) du dispositif d'éclairage (B) présente une atténuation par un facteur d'au moins 10⁶.

7. Système selon l'une des revendications 1 à 6, **caractérisé en ce que** le dispositif d'éclairage (B) comprend une coupole d'éclairage positionnable par un bras articulé.

8. Système selon l'une des revendications 1 à 6, **caractérisé en ce que** le dispositif d'éclairage (B) comprend une lampe frontale.

9. Système selon l'une des revendications 1 à 8, **caractérisé en ce que** le dispositif d'éclairage (B) est adapté pour fournir un éclairage continu.

10. Système selon l'une des revendications 1 à 9, **caractérisé en ce que** le dispositif d'éclairage est adapté pour fournir un éclairage pulsé.

11. Système selon l'une des revendications 1 à 10, **caractérisé en ce que** le dispositif d'éclairage (B) comprend des diodes électroluminescentes.

12. Système selon l'une des revendications 1 à 11, **caractérisé en ce que** la puissance du dispositif d'éclairage est supérieure ou égale à 40 kLux.

13. Système selon l'une des revendications 1 à 12, **caractérisé en ce que** le détecteur (D) est une caméra CCD ou CMOS.

14. Système selon l'une des revendications 1 à 13, **caractérisé en ce que** la longueur d'onde d'excitation est de 780 nm et **en ce que** le filtre (F_{D}) du détecteur transmet le rayonnement dans une bande comprise entre 820 et 850 nm.

15. Système selon l'une des revendications 1 à 14, **caractérisé en ce que** la longueur d'onde d'excitation est de 750 nm et **en ce que** le filtre (F_{D}) du détecteur transmet le rayonnement dans une bande comprise entre 780 et 870 nm.

## Patentansprüche

1. Fluoreszenzabbildungssystem für einen Operationssaal, das eine Vorrichtung (B) zur Beleuchtung des Operationssaals, die geeignet ist, ein weißes Licht zu emittieren, und eine Fluoreszenzabbildungsvorrichtung (I) umfasst,
wobei die Fluoreszenzabbildungsvorrichtung (I) Folgendes umfasst:
- eine Lichtquelle (S), die geeignet ist, eine Strahlung (L_{E}) zur Anregung eines fluoreszierenden Markers in einem Emissionswellenlängenbereich (L_{F}) zu emittieren, der zwischen 600 und 900 nm enthalten ist,
- einen Detektor (D), der angepasst ist, um die durch den Marker unter der Wirkung einer Anregung durch die Lichtquelle (S) emittierte Fluoreszenzstrahlung (L_{F}) zu detektieren,
- ein Filter (F_{D}) des Detektors (D), das ein Hochpass- oder Bandpassfilter (F_{D1}) umfasst, das angepasst ist, um die Anregungsstrahlung (L_{E}) zu blockieren und zum Detektor (D) die Photonen durchzulassen, deren Wellenlänge in dem Wellenlängenbereich der durch den Marker emittierten Fluoreszenzstrahlung (L_{F}) enthalten ist,
wobei das System ferner Folgendes umfasst
- ein Tiefpassfilter (F_{B}), das vor der Beleuchtungsvorrichtung (B) platziert ist und dessen Grenzwellenlänge (λ_{Bmax}) niedriger als der Emissionsbereich (L_{F}) des fluoreszierenden Markers ist, wobei das Tiefpassfilter (F_{B}) dennoch einen Anstieg oder Schwankungen der Dämpfung für Wellenlängen aufweisen kann, die höher als der Emissionsbereich (L_{F}) des fluoreszierenden Markers sind,
wobei das System **dadurch gekennzeichnet ist, dass** das Filter (F_{D}) des Detektors ein Tiefpassfilter (F_{D2}) umfasst, das mit dem Hochpass- oder Bandpassfilter (F_{D1}) kombiniert ist, derart, dass in einem Wellenlängenbereich (Z_{D}), der sich ausgehend von einer oberen Grenzwellenlänge (λₘₐₓ) erstreckt, die niedriger als die Wellenlänge ist, ab der ein Anstieg oder Schwankungen der Dämpfung des Tiefpassfilters der Beleuchtungsvorrichtung beobachtet werden, das Produkt der Dämpfung des Filters (F_{D}) des Detektors und der Dämpfung des Tiefpassfilters (F_{B}) der Beleuchtungsvorrichtung eine Dämpfung um einen Faktor von mindestens 10⁶ zur Folge hat.

2. System nach Anspruch 1, **dadurch gekennzeichnet, dass** die Wellenlänge der Anregungsstrahlung zwischen 630 und 810 nm enthalten ist.

3. System nach einem der Ansprüche 1 oder 2, **dadurch gekennzeichnet, dass** die Breite des Bandes, in dem das Filter des Detektors die Fluoreszenzstrahlung durchlässt, zwischen 50 und 70 nm enthalten ist.

4. System nach einem der Ansprüche 1 bis 3, **dadurch gekennzeichnet, dass** der Wellenlängenbereich (Z_{D}), in dem das Produkt der Dämpfung des Filters des Detektors und der Dämpfung des Tiefpassfilters der Beleuchtungsvorrichtung (B) eine Dämpfung um einen Faktor von mindestens 10⁶ zur Folge hat, sich mindestens bis zur Detektionsgrenzwellenlänge des Detektors (D) erstreckt.

5. System nach einem der Ansprüche 1 bis 4, **dadurch gekennzeichnet, dass** der Wellenlängenbereich, in dem das Produkt der Dämpfung des Filters des Detektors (D) und der Dämpfung des Tiefpassfilters der Beleuchtungsvorrichtung (B) eine Dämpfung um einen Faktor von mindestens 10₆ zur Folge hat, sich mindestens bis 1000 nm, vorzugsweise bis 1150 nm erstreckt.

6. System nach einem der Ansprüche 1 bis 5, **dadurch gekennzeichnet, dass** in dem Wellenlängenbereich (λ_{inf}, λₛᵤₚ), der durch das Filter des Detektors durchgelassen wird, das Tiefpassfilter (F_{B}) der Beleuchtungsvorrichtung (B) eine Dämpfung um einen Faktor von mindestens 10⁶ aufweist.

7. System nach einem der Ansprüche 1 bis 6, **dadurch gekennzeichnet, dass** die Beleuchtungsvorrichtung (B) eine Beleuchtungskuppel umfasst, die durch einen Gelenkarm positionierbar ist.

8. System nach einem der Ansprüche 1 bis 6, **dadurch gekennzeichnet, dass** die Beleuchtungsvorrichtung (B) eine Stirnlampe umfasst.

9. System nach einem der Ansprüche 1 bis 8, **dadurch gekennzeichnet, dass** die Beleuchtungsvorrichtung (B) angepasst ist, um eine ununterbrochene Beleuchtung zu liefern.

10. System nach einem der Ansprüche 1 bis 9, **dadurch gekennzeichnet, dass** die Beleuchtungsvorrichtung angepasst ist, um eine pulsierende Beleuchtung zu liefern.

11. System nach einem der Ansprüche 1 bis 10, **dadurch gekennzeichnet, dass** die Beleuchtungsvorrichtung (B) lichtemittierende Dioden umfasst.

12. System nach einem der Ansprüche 1 bis 11, **dadurch gekennzeichnet, dass** die Leistung der Beleuchtungsvorrichtung höher oder gleich 40 kLux beträgt.

13. System nach einem der Ansprüche 1 bis 12, **dadurch gekennzeichnet, dass** der Detektor (D) eine CCD- oder CMOS-Kamera ist.

14. System nach einem der Ansprüche 1 bis 13, **dadurch gekennzeichnet, dass** die Anregungswellenlänge 780 nm beträgt, und dadurch, dass das Filter (F_{D}) des Detektors die Strahlung in einem Band durchlässt, das zwischen 820 und 850 nm enthalten ist.

15. System nach einem der Ansprüche 1 bis 14, **dadurch gekennzeichnet, dass** die Anregungswellenlänge 750 nm beträgt, und dadurch, dass das Filter (F_{D}) des Detektors die Strahlung in einem Band durchlässt, das zwischen 780 und 870 nm enthalten ist.

## Claims

1. A fluorescence imaging system for an operating room comprising an operating room illuminating device (B) capable of emitting white light and a fluorescence imaging device (I),
said fluorescence imaging device (I) comprising:
- a light source (S) capable of emitting excitation radiation (L_{E}) to excite a fluorescent marker in a range of emission wavelengths (L_{F}) of between 600 and 900 nm,
- a detector (D) adapted to detect the fluorescent radiation (L_{F}) emitted by said marker under the effect of excitation by said light source (S),
- a filter (F_{D}) of the detector (D) comprising a band-pass or high-pass filter (F_{D1}) adapted to block the excitation radiation (L_{E}) and to transmit to the detector (D) those photons having a wavelength included in the range of wavelengths of fluorescent radiation (L_{F}) emitted by said marker,
the system further comprising
- a low-pass filter (F_{B}) placed in front of the illuminating device (B) and having a cut-off wavelength (λ_{Bmax}) below the emission range (L_{F}) of the fluorescent marker, said low-pass filter (F_{B}) nonetheless being able to exhibit attenuation peaking or fluctuations at wavelengths above the emission range (L_{F}) of the fluorescent marker,
the system being **characterized in that** the filter (F_{D}) of the detector comprises a low-pass filter (F_{D2}) combined with the band-pass or high-pass filter (F_{D1}) so that, in a range of wavelengths (Z_{D}) extending from an upper cut-off wavelength (λₘₐₓ) below the wavelength on and after which attenuation peaking or fluctuations of the low-pass filter of the illuminating device are observed, the product of attenuation by the filter (F_{D}) of the detector and of attenuation by the low-pass filter (F_{B}) of the illuminating device leads to an attenuation by a factor of at least 10⁶.

2. The system according to claim 1, **characterized in that** the wavelength of excitation radiation is between 630 and 810 nm.

3. The system according to one of claims 1 or 2, **characterized in that** the bandwidth in which the filter of the detector transmits fluorescent radiation is between 50 and 70 nm.

4. The system according to one of claims 1 to 3 **characterized in that** the range of wavelengths (Z_{D}), in which the product of attenuation by the detector filter and of attenuation by the low-pass filter of the illuminating device (B) leads to attenuation by a factor of at least 10⁶, extends at least as far as the limit detection wavelength of the detector (D).

5. The system according to one of claims 1 to 4 **characterized in that** the range of wavelengths, in which the product of attenuation by the filter of the detector (D) and of attenuation by the low-pass filter of the illuminating device (B) leads to attenuation by a factor of at least 10⁶, extends at least as far as 1000 nm, preferably as far as 1150 nm.

6. The system according to one of claims 1 to 5 **characterized in that** in the range (λ_{lower}, λᵤₚₚₑᵣ) of wavelengths transmitted by the detector filter, the low-pass filter (F_{B}) of the illuminating device (B) exhibits attenuation by a factor of at least 10⁶.

7. The system according to one of claims 1 to 6, **characterized in that** the illuminating device (B) comprises a dome-shaped light-head which can be positioned via a hinged arm.

8. The system according to one of claims 1 to 6, **characterized in that** the illuminating device (B) comprises a headlamp.

9. The system according to one of claims 1 to 8, **characterized in that** the illuminating device (B) is adapted to provide continuous lighting.

10. The system according to one of claims 1 to 9, **characterized in that** the illuminating device is adapted to provide pulsed lighting.

11. The system according to one of claims 1 to 10, **characterized in that** the illuminating device (B) comprises light-emitting diodes.

12. The system according to one of claims 1 to 11, **characterized in that** the power of the illuminating device is 40 kLux or higher.

13. The system according to one of claims 1 to 12, **characterized in that** the detector (D) is a CCD or CMOS camera.

14. The system according to one of claims 1 to 13, **characterized in that** the excitation wavelength is 780 nm and **in that** the filter (F_{D}) of the detector transmits radiation in a band between 820 and 850 nm.

15. The system according to one of claims 1 to 14, **characterized in that** the excitation wavelength is 750 nm and **in that** the filter (F_{D}) of the detector transmits radiation in a band between 780 and 870 nm.
